(19) Europäisches Patentamt
European Patent Office
Office européen des brevets

(11) Veröffentlichungsnummer: **0 248 209**
**A2**

(12) **EUROPÄISCHE PATENTANMELDUNG**

(21) Anmeldenummer: **87106224.6**

(22) Anmeldetag: **29.04.87**

(51) Int. Cl.³: **C 07 K 7/64**
A 61 K 37/02
//C07K7/06

(30) Priorität: **02.05.86 DE 3614904**
**03.02.87 DE 3703159**

(43) Veröffentlichungstag der Anmeldung:
**09.12.87 Patentblatt 87/50**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kessler, Horst, Prof. Dr.**
**Friedrich-Stoltze-Strasse 53**
**D-6231 Schwalbach(DE)**

(72) Erfinder: **Haupt, Andreas, Dr.**
**Karlsbader Strasse 13**
**D-6370 Oberursel(DE)**

(72) Erfinder: **Schudok, Manfred**
**Birminghamstrasse 89**
**D-6230 Frankfurt am Main 80(DE)**

(54) **Cyclische Peptide mit cytoprotektiver Wirkung.**

(57) Beschrieben werden cyclische Hexapeptide der allgemeinen Formel I, cyclische dimere Hexapeptide der allgemeinen Formel II

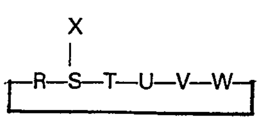

(I)

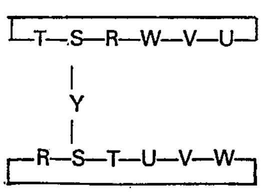

(II)

und die entsprechenden offenkettigen Hexapeptide sowie Verfahren zu deren Herstellung und die Verwendung der Verbindungen der Formeln I und II.

EP 0 248 209 A2

## Cyclische Peptide mit cytoprotektiver Wirkung

Die Erfindung betrifft cyclische Hexapeptide der allgemeinen Formel I und cyclische dimere Hexapeptide der allgemeinen Formel II

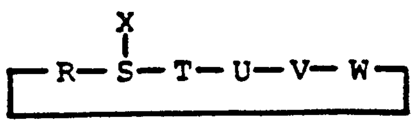

(I)

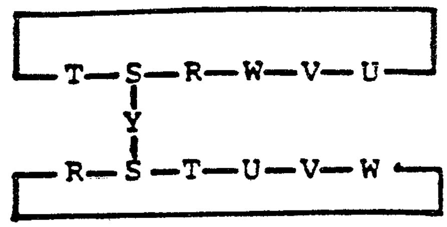

(II)

in der

R Thr, Val, Ala, Phe oder Thr, das gegebenenfalls verestert sein kann; T Trp;

S Lys, Orn, $C{\equiv}C-CH_2-NH_2$, $CH_2-C{\equiv}C-CH_2-NH_2$, $CO-(CH_2)_2-CO-O-(CH_2-CH_2-O)_m-CH_3$ oder $CO-(CH_2)_2-CO-O-(CH_2-CH_2-O)_m-H$;

U Phe, Phe(p-NHZ) oder Tyr, das ggf. verestert sein kann;

V D-Pro oder D-Ala;

W Phe oder Phe(p-NHZ);

X H, Z, $CO-C_6H_5$, $CO-C_6H_4(p-N_3)$ oder $CO-(CH_2)_n-C_6H_4(p-OSO_3Na)$;

Y $CO-(CH_2)_n-CO$, wobei max. 3 $CH_2$-Gruppen durch O ersetzt sein können, CO, $CO-C_6H_4(p-CO)$, $CO-CH_2-O-(CH_2-CH_2-O-)_m-CH_2-CO$ oder $CO-C{\equiv}C-CO$, $CO-(CH{=}CH)_n-CO$;

Z Benzyloxycarbonyl;

m eine ganze Zahl von 1 - 15 und

n eine ganze Zahl von 1 - 16

bedeuten,

sowie deren physiologisch verträglichen Salze.

Bevorzugt sind diejenigen Verbindungen der Formel I und II in denen mindestens einer der Substituenten bzw. Indices die folgende Bedeutung hat:

R Val oder $Thr(OSO_3-Na)$,

U $Tyr(OSO_3Na)$,

Y $CO-(CH_2)_n-CO$, $CO-CH_2-O-(CH_2-CH_2-O-)_m$ $CH_2-CO$ oder $CO-C{\equiv}C-CO$,

m eine ganze Zahl von 10 - 12 und

n 4.

Verbindungen der Formel I, welche eine freie Aminogruppe enthalten, bilden Salze mit anorganischen Säuren, wie z.B. Salzsäure, Schwefelsäure oder Phosphorsäure und mit organischen Carbon- oder Sulfonsäure, wie z.B. Essigsäure, Citronensäure, Benzoesäure, Maleinsäure, Fumarsäure, Weinsäure und p-Toluolsulfonsäure.

Die Erfindung betrifft weiterhin ein Verfahren zur Herstellung einer Verbindung der Formel I und Formel II, das dadurch gekennzeichnet ist, daß man ein lineares Hexapeptid einer der allgemeinen Formeln II a - II f

| | |
|---|---|
| $H - R - S(R^1) - T - U - V - W - OH$ | (II a) |
| $H - S(R^1) - T - U - V - W - R - OH$ | (II b) |
| $H - T - U - V - W - R - S(R^1) - OH$ | (II c) |
| $H - U - V - W - R - S(R^1) - T - OH$ | (II d) |
| $H - V - W - R - S(R^1) - T - U - OH$ | (II e) |
| $H - W - R - S(R^1) - T - U - V - OH$ | (II f) |

in denen R, S, T, U, V und W vorstehendene Bedeutungen haben und $R^1$ für eine Schutzgruppe der $\delta$ - oder $\varepsilon$ -Aminofunktion steht, nach bekannten Verfahren der Peptidsynthese cyclisiert, temporär eingeführte Schutzgruppen in geeigneter Weise abspaltet und anschließend zur Herstellung einer Verbindung der Formel II zwei solcherart erhaltene ungeschützte dicyclische Hexapeptide der Formel I über eine Gruppe Y dimerisiert und die so erhaltenen Peptide der Formel I oder II gegebenenfalls in ihre physiologisch verträglichen Salze überführt (Wünsch et al., Houben-Weyl, Bd 15/1,2 Stuttgart: Thieme 1974; Bodansky et al., The Practice of Peptide Synthesis, Springer, New York, 1984).

Eine einfache Verfahrensvariante ist z.B. dadurch gekennzeichnet, daß man ein lineares Peptidderivat einer der Formeln III a - III f

Boc - R - S($R^1$) - T - U - V - W - $NH-NH_2$ (III a)
Boc - S($R^1$) - T - U - V - W - R - $NH-NH_2$ (III b)
Boc - T - U - V - W - R - S($R^1$) - $NH-NH_2$ (III c)
Boc - U - V - W - R - S($R^1$) - T - $NH-NH_2$ (III d)
Boc - V - W - R - S($R^1$) - T - U - $NH-NH_2$ (III e)
Boc - W - R - S($R^1$) - T - U - V - $NH-NH_2$ (III f)

worin R, S, T, U, V, W und $R^1$ wie oben definiert sind, nach Abspaltung der Boc-Gruppe über in situ hergestelltes Azid cyclisiert und anschließend die restlichen temporär eingeführten Schutzgruppen entfernt.

Die Erfindung betrifft auch allgemein Verbindungen der Formeln IV a - IV f

$R^2$ - R - S($R^4$) - T - U - V - W - $R^3$ (IV a)
$R^2$ - S($R^4$) - T - U - V - W - R - $R^3$ (IV b)
$R^2$ - T - U - V - W - R - S($R^4$) - $R^3$ (IV c)
$R^2$ - U - V - W - R - S($R^4$) - T - $R^3$ (IV d)
$R^2$ - V - W - R - S($R^4$) - T - U - $R^3$ (IV e)
$R^2$ - W - R - S($R^4$) - T - U - V - $R^3$ (IV f)

in denen R, S, T, U, V und W vorstehende Bedeutung haben,
$R^2$ H, Z, Fmoc oder Boc,
$R^3$ OH oder $NH-NH_2$ bedeuten und
$R^4$ im Falle $R^2$ = H für Z oder Boc,
im Falle $R^2$ = Z oder Fmoc für Boc oder
im Falle $R^2$ = Boc für Z steht.

Die linearen Peptide der Formeln IVa - IVf können sowohl klassich (Wünsch et al., Houben-Weyl, Bd 15/1,2 Stuttgart: Thieme 1974; Bodansky et al., The Practice of Peptide Synthesis, Springer, New York, 1984) als auch mittels Festphasensynthese (z.B. E. Wünsch, Angew. Chem. 83 (1971) 773; Felix und Merrifield, J. Am. Chem. Soc. 92 (1970) 1385) hergestellt werden. Bei der Festphasensynthese (wie auch bei der klassischen) ist es für das cyclische Endprodukt unwesentlich, welche Aminosäure C-terminal steht, da dies nach der Cyclisierung nicht mehr feststellbar ist.

Bei der klassischen Peptidsynthese dient beispielsweise der durch katalytische Hydrierung abspaltbare Z-Rest oder der durch sekundäre Amine abspaltbare 9-Fluorenylmethyloxycarbonylrest (Fmoc) als Aminoschutzgruppe, während die δ-Aminogruppe des Ornithins oder die ε-Aminogruppe des Lysins vorzugsweise durch den Boc-Rest geschützt wird. Die Peptide der allgemeinen Formel IV a - f können stufenweise oder über vorgefertigte Segmente aufgebaut werden.

Folgende lineare Vorläufer eines erfindungsgemäßen Peptids der Formel I führen z.B. alle zum gleichen Endprodukt:

Boc-Thr-Lys(Z)-Trp-Phe-D-Pro-Phe-$NHNH_2$
Boc-Lys(Z)-Trp-Phe-D-Pro-Phe-Thr-$NHNH_2$
Boc-Trp-Phe-D-Pro-Phe-Thr-Lys(Z)-$NHNH_2$
Boc-Phe-D-Pro-Phe-Thr-Lys(Z)-Trp-$NHNH_2$
Boc-D-Pro-Phe-Thr-Lys(Z)-Trp-Phe-$NHNH_2$
Boc-Phe-Thr-Lys(Z)-Trp-Phe-D-Pro-$NHNH_2$

Tryptophan nicht allzu früh in die Kette einzufügen, hat den Vorteil, daß es bei Abspaltung der Boc-Schutzgruppen weniger oft der Gefahr der tert.-Butylierung ausgesetzt ist. Wenn Threonin direkt an den polymeren Träger angekuppelt werden soll, muß die OH-Funktion geschützt werden (z.B. als Benzylether), um Nebenreaktionen zu vermeiden.

Steht Threonin jedoch an zweiter bis sechster Stelle in der linearen Peptidkette, kann in der Regel auf eine OH-Schutzgruppe verzichtet werden.

Die Synthese der linearen Vorläufer erfolgt z.B. schrittweise an hydroxymethyliertem Polystyrol-Harz. Das Polystyrol ist mit beispielsweise 1 % Divinylbenzol quervernetzt. Es liegt gewöhnlich in Form kleiner Kügelchen vor.

Die Aminosäuren werden N-terminal geschützt eingesetzt. Die erste N-geschützte Aminosäure wird per Esterbildung am Träger angebracht. Nach Beseitigung der Aminoschutzgruppe wird die nächste N-geschützte Aminosäure unter Verwendung eines Kupplungsreagenzes wie Dicyclohexylcarbodiimid angeknüpft. Entschützen und Zufügen weiterer Aminosäuren wird fortgesetzt, bis der gewünschte lineare Vorläufer erreicht ist.

Die Auswahl der Schutzgruppen richtet sich nach den Aminosäuren und Kupplungsmethoden.

Als Aminoschutzgruppen kommen z.B. die bekannten urethanischen Schutzgruppen wie Benzyloxycarbonyl (Z), p-Methoxycarbobenzoxy , p-Nitrocarbobenzoxy , t-Butyloxycarbonyl (Boc) und ähnliche in Frage.

Die Boc-Gruppe wird bevorzugt, da sie durch relativ milde Säuren (z.B. Trifluoressigsäure oder HCl in organischen Lösungsmitteln) abspaltbar ist.

Threonin kann, wie bereits erwähnt, als Benzylether blockiert werden und die δ-Aminofunktion des Ornithins und die ε-Aminofunktion des Lysins als Z-Derivat. Diese beiden Schutzgruppen sind gegen die Abspaltungsreagenzien für die Boc-Gruppe weitestgehend resistent und können nach der Cyclisierung hydrogenolytisch mit einem Hydrierkatalysator (Pd/Aktivkohle) oder z.B. mit Natrium in flüssigem Ammoniak entfernt werden.

Das lineare geschützte Peptid kann mit Hydrazin vom Harz genommen werden. Dabei entsteht das Hydrazid, welches mit einem Reagenz, das in situ salpetrige Säure freisetzt, in das Azid überführt werden kann. Geeignete Reagenzien stellen niedere Alkylnitrite (z.B. t-Butylnitrit, Isoamylnitrit) oder Alkalinitrite (z.B. Natriumnitrit, Kaliumnitrit) in gegenwart starker Säuren wie Salzsäure, Phosphorsäure, Sulfonsäuren usw. dar. Diese Umsetzung kann in verschiedenen Lösungsmitteln wie DMF, THF, Dioxan, Methylenchlorid, Chloroform bei Temperaturen zwischen $-20^{\circ}$ und $+30^{\circ}C$, bevorzugt zwischen $-15^{\circ}$ und $+10^{\circ}C$, erfolgen. Die saure Azidlösung wird verdünnt und durch Zugabe von Base neutralisiert. Das lineare Peptid cyclisiert zum cyclischen, geschützten Hexapeptid.

Das Hydrazid kann jedoch auch z.B. mit N-Bromsuccinimid nach Int. J. Pept. Prot. Research 17 (1981) 6 - 11 in die freie Carbonsäure überführt werden. Dann ist nach dem Deblockieren des N-terminalen Endes eine Cyclisierung mit Reagenzien wie DCC möglich.

Nach der Cyclisierung werden die restlichen temporär eingeführten Schutzgruppen beseitigt. Diese cyclischen ungeschützten Peptide der Formel I stellen die Bausteine für die Herstellung einer Verbindung der Formel II dar.

Die Reinigung der Cyclisierungsrohprodukte erfolgt vorzugsweise chromatographisch, insbesondere durch Gelfiltration (beispielsweise an Sephadex $^{(R)}$ / DMF).

Das gereinigte ungeschützte cyclische Peptid der Formel I kann nun mit dem Anhydrid einer Dicarbonsäure - beispielsweise Bernsteinsäureanhydrid - zur Reaktion gebracht werden. Weitere Umsetzung des so erhaltenen modifizierten Peptides mit anderen cyclischen ungeschützten Peptiden der Formel I unter Zuhilfenahme von Kondensationsmitteln wie DCC oder EDCI liefert dimere Verbindungen der Formel II mit Y= $CO(CH_2)_nCO$.

Dimere der Formel II mit Y= $CO-CH_2-O-(CH_2-CH_2-O-)_m-CH_2-CO$ werden beispielsweise durch Dimerisierung eines ungeschützten Peptids der Formel I mit der Disäure $HO-OC-CH_2-O-(CH_2-CH_2-O)_m-CH_2-COOH$ (erhältlich aus dem entsprechenden Glykol oder Polyglykol durch Oxidation) erhalten.

Weiterhin kann man das ungeschützte cyclische Peptid der Formel I mit Reagenzien, welche die Gruppen Z, $COC_6H_5$ oder $COC_6H_4(p-N_3)$ übertragen (z.B. Benzyloxycarbonylchlorid, Benzoylchlorid oder p-Azidobenzoylchlorid), in Gegenwart von Basen in die entsprechenden geschützten Peptide der Formel I überführen.

Die Reinigung der Produkte erfolgt vorzugsweise durch Gelfiltration (beispielsweise an Sephadex $^{(R)}$ / DMF).

Die Erfindung betrifft ferner die Verwendung der Verbindungen der Formeln I und II als Heilmittel, pharmazeutische Präparate, die diese Verbindungen enthalten, Verfahren zu deren Herstellung und deren Verwendung als Heilmittel.

Die erfindungsgemäßen cyclischen Hexapeptide der Formel I und cyclischen dimeren Hexapeptide der Formel II enthalten die Retro-Sequenz eines Bereiches der Formel V von Somatostatin (VI), der für die biologische Wirkung essentiell ist.

- Phe - Trp - Lys - Thr - (V)

H-Ala-Gly-Cys-Lys-Asn-Phe-Phe-Trp-Lys-Thr-Phe-Thr-Ser-Cys-OH

(VI)

Das Tetrapeptid V ist für sich allein unwirksam. Erst wenn es in eine somatostatinähnliche Konformation gebracht wird,

z.B. durch Cyclisierung über ein geeignetes Dipeptid zu einem Cyclohexapeptid der Formel VII analog Nature 292, 55 - 58 (1981) oder durch Cyclisierung über eine Disulfidbrücke zu einem heterodet cyclischen Peptid der Formel VIII analog Life Sci. 31, 1133 - 1140 (1982), erscheint die biologische Wirkung.

Pro - Phe - D-Trp
Phe - Thr - Lys          (VII)

D-Phe-Cys-Phe-D-Trp-Lys-Thr-Cys-Thr(ol          (VIII)

Somatostatin und Analoga der Formeln VII und VIII inhibieren die Ausschüttung zahlreicher Peptid- und Proteohormone.

Somatostatin schützt auch verschiedene Gewebe, z.B. Darmschleimhaut oder Leberzellen gegen unterschiedliche Noxen. Hier besteht eine Analogie zur protektiven Wirkung der Prostaglandine. Der Intoxikation durch z.B. Cysteamin, Alkohol oder Phalloidin steht eine "membranstabilisierende Wirkung" des Somatostatins gegenüber.

Läsionen von Rattenleberzellen, die experimentell durch Zellgifte hervorgerufen werden, konnten bei gleichzeitiger Gabe oder nach Vorbehandlung mit Somatostatin beträchtlich reduziert werden.

Überraschenderweise üben nun die erfindungsgemäßen Verbindungen der Formeln I und II, die die abgewandelte retro-Sequenz des Somatostatins 6-11 enthalten, eine stärkere cytoprotektive Wirkung aus als Somatostatin, während die für Somatostatine charakteristische, aber häufig unerwünschte Inhibierung der Hormonfreisetzung nicht vorhanden ist.

Zur Prüfung der cytoprotektiven Wirkung wurde folgendes Verfahren eingesetzt:

Hemmung der Cholat- und Phalloidinaufnahme an isolierten Rattenleberzellen

Somatostatin und hier beschriebene Analoga inhibieren die Aufnahme von Phalloidin und Cholsäure in isolierte Rattenleberzellen. Phalloidin- und Cholat-Aufnahmehemmung korrelieren dabei in eindeutiger Weise. Isolierte Hepatocyten wurden nach der Methode von Berry und Friend aus männlichen Wistar Ratten gewonnen. Alle Experimente wurden innerhalb von 2 Stunden nach Zellisolation durchgeführt. Der Zellvitalitätstest wurde mit Trypan-Blau durchgeführt: 85 - 90 % aller Zellen zeigten keinen Eintritt des Farbstoffs ins Zellinnere. Zur Messung der Aufnahmehemmung wurden 1 ml Hepatocytensuspension ($2 \times 10^6$ Zellen/ml, entsprechend 4 mg Zellprotein) 30 Sekunden lang mit verschiedenen Konzentrationen der zu testenden Substanzen inkubiert. 15, 45, 75, 105, 135 Sekunden, 5 und 10 Minuten nach Zugabe von 1 $\mu$M $^{14}$C-Cholsäure plus 6 $\mu$M Cholat oder 0,1 $\mu$M $^3$H-DMP plus 6 $\mu$M Phalloidin wurden jeweils 100 $\mu$l entnommen und analysiert. Zellassoziiertes radioaktives Substrat wurde von freiem Liganden mit der Silikonöl-Mikrozentrifugen-Methode abgetrennt. Die Radioaktivität im Zellpellet wurde in Lipoluma/Lumasolve/Wasser (100/10/2 v/v) in einem Szintillationszähler bestimmt. Es wurden diejenigen Konzentrationen der getesteten Substanzen ermittelt, die eine 50 % ige Hemmung der Phalloidin- bzw. Cholataufnahme bedingen.

| Verbindung | cytoprotektive Dosis $_{50}$ ($\mu$M) Phalloidin | Cholat |
|---|---|---|
| Somatostatin | 109 | |
| c-(-D-Pro-Phe-Thr-Lys(Z)-Trp-Phe-) | 1 | 1,5 |
| c-(-D-Pro-Phe-Thr-Lys-Trp-Phe-) | 1,5 | 3 |
| c-(-D-Pro-Phe-Thr-Lys(CO($CH_2$)$_2$COOH)-Trp-Phe-) | | 9 |
| 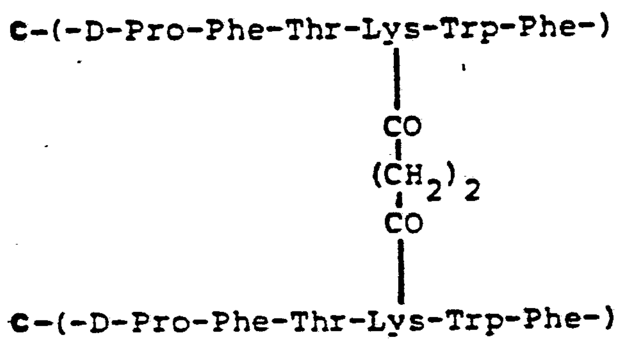 | | 0,14 - 0,18 |

50 %ige Hemmung der Cholataufnahme in isolierten Ratten-Hepatocyten:

| Verbindung | IC 50 (µM) |
|---|---|
| (Orn$^{9}$)- A (Z) | 2,0 |
| (Orn$^{9}$)- A | 8,0 |
| (Orn$^{9}$)- A - CO - A | 0,045 |
| A - CO - A | 1,15 |
| A - Malonsäure - A | 0,018 |
| A - Adipinsäure - A | 0,011 |
| A - Azelainsäure - A | 0,034 |
| A - Dodekandisäure - A | 0,6 |
| A - Hexadekandisäure - A | 0,05 |
| A - Trioxaundekandisäure - A | 0,025 |
| A - Polyglykoldisäure - A | 0,7 |
| A - Terephtalsäure - A | 0,22 |
| A - Acetylendicarbonsäure - A | 0,001 |
| (Thr$^{10}$ - Tyr$^{7}$)- A (Z)<br>$OSO_3Na$ $OSO_3Na$ | 2,2 |

$A = c(-Trp - Phe^{7} - D\text{-}Pro - Phe - Thr - Lys^{9}-)$

Die neuen Peptide können als Arzneimittel intravenös, subcutan, intranasal, sublingual oder peroral eingesetzt werden. Sie sind besonders in der Behandlung von akuten Läsionen der Leber, des exokrinen Pankreas oder des Verdauungstraktes von großem Wert.

Wegen der lang anhaltenden Wirkung reichen bei einem normalgewichtigen Erwachsenen täglich 2 - 3 subcutane Injektionen von je 0,05 - 10 mg zur Behandlung der genannten Erkrankungen aus. Sublingual ist mit der 5 - 50-fachen Dosierung zu rechnen, peroral mit der 10 - 200-fachen Dosierung. Bei der intranasalen Applikation sollte die 5 - 50-fache Menge der jeweils subcutan wirksamen Dosis angewandt werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfstoffe in Lösung, Suspension oder Emulsion gebracht. Als Lösungsmittel für die neuen aktiven Verbindungen und die entsprechend physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose- oder Mannitlösungen, oder auch eine Mischung aus den verschiedenen genannten Lösungsmitteln.

Die Anwendung kann auch durch kontinuierliche intravenöse Infusion (wie z.B. Dauerinfusion, externe oder implantierte automatische Dosiervorrichtungen) erfolgen.

Das folgende Synthesebeispiel dient ausschließlich der Illustration und stellt keine Beschränkung der oben genannten Methoden dar.

Im folgenden verwendete Abkürzungen:

| | |
|---|---|
| AS | Aminosäure |
| DCC | Dicyclohexylcarbodiimid |
| DMF | Dimethylformamid |
| DIPEA | Diisopropylethylamin |
| DMAP | 4-Dimethylaminopyridin |
| EDCI | 1-Ethyl-3-(3-dimethylaminopropyl)-carbodiimid-hydrochlorid |
| HOBT | 1-Hydroxybenzotriazol |
| MeOH | Methanol |
| MSA | Methansulfonsäure |
| TFA | Trifluoressigsäure |

Laufmittel für die Dünnschichtchromatographie

| | | |
|---|---|---|
| A | n-Butanol/Essigsäure/Wasser | 3/1/1 |
| B | $CHCl_3$/MeOH/Essigsäure | 95/5/3 |
| C | $CHCl_3$/MeOH/Essigsäure | 80/20/3 |

Boc-Trp-Phe-D-Pro-Phe-Thr-Lys(Z)-$N_2H_3$

Die Herstellung des linearen Hexapeptides erfolgt am polymeren Träger (Merrifield-Synthese).

Vorbereitung des polymeren Trägers:

Mit 1 % Divinylbenzol quervernetztes Chloromethylpolystyrol wird in bekannter Weise in das gewünschte Hydroxymethylpolystyrol überführt.

Man setzt zunächst das Chloromethylpolystyrol mit Kaliumacetat in Dimethyacetamid um. Das acetylierte Merrifieldharz wird dann einer Hydrazinolyse in DMF unterworfen. Die Umsetzungen verlaufen quantitativ.

Ankondensieren der ersten Aminosäure an das Harz:

Mit Methylenchlorid gewaschenes und vorgequollenes Hydroxymethylpolystyrol (4 g), 3,8 g (10 mmol) Boc-Lys(Z)-OH, 2,1 g (10 mmol) DCC, 1,23 g (10 mmol) DMAP werden in 60 ml $CH_2Cl_2$ in einem Schüttelgefäß aufgeschlämmt. Die Mischung wird bei Raumtemperatur 5 h geschüttelt. Die Aufarbeitung ist im nachstehenden Fließschema dargestellt.

Noch freie Hydroxylgruppen werden durch Veresterung mit Benzoylchlorid unter Zusatz von Pyridin in Methylenchlorid blockiert.

Die Abspaltung der Boc-Schutzgruppe wird mit TFA/MSA in Methylenchlorid durchgeführt. Nach der Neutralisation liefert die Pikratbestimmung einen Wert (z.B.: 2,74 mmol/4 g Harz) für den Gehalt an freiem Amin.

Fließschema zur Festphasensynthese

| Stufe | Anzahl | Zeit (min) | Menge (ml) | Substanzen |
|---|---|---|---|---|
| 1 | 3 | 3 | 60 | $CH_2Cl_2$, Harz |
| 2 | 1 | 5 h | 60 | " " DMAP, Boc-AS, DCC |
| 3 | 4 | 2 | 50 | $CH_2Cl_2$/MeOH 1:1 |
| 4 | 2 | 2 | 50 | $CH_2Cl_2$ |
| 5 | 1 | 20 | 50 | " , 0,4 ml Pyridin, 0,5 ml Benzoylchlorid |
| 6 | 5 | 2 | 50 | $CH_2Cl_2$ |
| 7 | 3 | 10 | 40 | 10,3 ml TFA, 0,52 ml MSA, 110 ml $CH_2Cl_2$ |
| 8 | 2 | 3 | 50 | $CH_2Cl_2$/Dioxan 1:1 |
| 9 | 2 | 3 | 50 | $CH_2Cl_2$/MeOH 1:1 |
| 10 | 2 | 3 | 50 | $CH_2Cl_2$ |

| | | | | |
|---|---|---|---|---|
| 11 | 2 | 5 | 50 | 25 ml DIPEA, 125 ml $CH_2Cl_2$ |
| 12 | 5 | 2 | 50 | $CH_2Cl_2$ |
| 13 | 1 | | 60 | " , 8 mmol Boc-AS, DCC, HOBT |
| 14 | 4 | 2 | 50 | $CH_2Cl_2$/MeOH 1:1 |
| 15 | 2 | 2 | 50 | $CH_2Cl_2$ |
| Rapport ab Stufe 7 | | | | |

Verlängerung der Peptidkette am Harz:

Die zweite und alle weiteren Boc-Aminosäuren (8 mmol), werden mit äquimolaren Mengen DCC und HOBT in Methylenchlorid angekuppelt. Das Ende der Umsetzung wird durch den sogenannten Chloraniltest (Chloranil in Toluol, Acetanhydrid, Harz aus der Reaktionsmischung) ermittelt. Die Reaktionszeiten variieren zwischen 0,5 h und ca. 15 h.

Abnehmen des linearen Vorläufers vom Polymer:

Nach Anknüpfen der letzteren Aminosäure unterbleibt die Schutzgruppenabspaltung. Sind die überschüssigen Reaktionspartner und auch der Dicyclohexylharnstoff ausgewaschen, wird das Peptidharz auf der Fritte scharf trockengesaugt, in DMF suspendiert und mit 5 ml absolutem Hydrazin versetzt. Man rührt über's Wochenende bei Raumtemperatur. Der lineare Vorläufer liegt nun als Hydrazid in DMF gelöst vor. Das Harz wird abfiltriert und die Lösung zur Trockene eingeengt. Der feste Rückstand wird mit Wasser digeriert. Boc-Thr-Lys(Z)-Trp-Phe-Phe-Phe-$NHNH_2$ geht nicht in Lösung. Der Feststoff wird abgenutscht und im Exsikkator über Phosphorpentoxid getrocknet.

Ausbeute: 2,71 g = 90 % d. Th. bezgl. Boc-Lys(Z)-Harz
= 62,5 % d. Th. bezgl. Cl-Harz

Smp.: 82 - 85°C unter Zersetzung

Entfernung der Boc-Schutzgruppe

2 mmol (2,18 g) Boc-Hexapeptid-Hydrazid werden in 9 ml absoluter TFA suspendiert und bei Raumtemperatur 20 Minuten gerührt. Man gibt 100 ml absoluten Diethylether dazu und saugt den ausfallenden weißen Feststoff über eine Fritte ab. Nach mehrmaligem Waschen mit Diethylether wird über $P_4O_{10}$ im Exsikkator getrocknet. Es handelt sich hierbei um TFA · H-Trp-Phe-D-Pro-Phe-Thr-Lys(Z)-$N_2H_3$ · TFA. Die Ausbeute ist quantitativ.

Cyclisierung zu cyclo (-D-Pro-Phe-Thr-Lys(Z)-Trp-Phe-)

Die Cyclisierung wird nach der Azidmethode durchgeführt. 2 mmol H-Trp-Phe-D-Pro-Phe-Thr-Lys(Z)-$N_2H_3$ · 2 TFA werden in 15 ml DMF gelöst, auf -18°C gekühlt, mit 10 mmol konz. HCl (36 %ig, 0,8 ml) und 3 mmol Isopentylnitrit (0,36 ml) versetzt. Das Azid bildet sich bei -8°C bis -10°C. Der Ansatz wird in 2 l -20°C kaltes DMF überführt und mit 3,1 ml DIPEA neutralisiert. Es wird noch drei Stunden bei tiefer Temperatur gerührt. Der gesamte Ansatz wird 3 Tage bei +2°C bis +10°C belassen. Der nach dem Einengen verbleibende ölige Rückstand wird über Nacht in Methanol/Wasser mit Mischbettionenaustauscher gerührt. Das Rohprodukt wird an Sephadex LH20 / DMF chromatographiert.

Ausbeute: 940 mg = 50 % d. Th.
Smp.: 135 - 145 °C
DC: $R_{fA}$ 0,89 $R_{fB}$ 0,10 $R_{fC}$ 0,86
FAB-MS $(M+H)^+$: 941
AS-Analyse: Pro 1,00 Thr 0,91 Phe 2,09 Lys 1,00

Entfernung der Z-Schutzgruppe

300 mg geschütztes cyclisches Hexapeptid werden unter Zusatz von 140 mg Katalysator (Pd/Aktivkohle, 10 %ig) und 300 mg Ammoniumformiat in MeOH als Lösungsmittel hydrogenolytisch in die entsprechende Verbindung mit freier

Seitenketten-Aminogruppe überführt. Nach Beendigung der Reaktion wird vom Katalysator abfiltriert, eingeengt und getrocknet.

Ausbeute: quantitativ
Smp.: 170 - 175 °C
DC: $R_{fB}$ 0,01 $R_{fC}$ 0,15

Umsetzung mit Bernsteinsäureanhydrid

100 mg des entschützten cyclischen Peptides (125 µmol) werden in wenig DMF gelöst und mit 50 mg Bernsteinsäureanhydrid (500 µmol) versetzt. Man rührt über Nacht bei Raumtemperatur, engt zur Trockne ein und versetzt mit wenig Wasser. Das modifizierte Peptid cyclo-(-D-Pro-Phe-Thr-Lys($CO(CH_2)_2COOH$)-Trp-Phe-) fällt aus und wird auf einer Fritte gesammelt, mehrmals mit Wasser gewaschen und im Exsikkator über $P_4O_{10}$ getrocknet.

Ausbeute: 90,6 mg = 80 % d.Th.
Smp.: 137 - 142 °C
DC: $R_{fB}$ 0,00 $R_{fC}$ 0,75

Dimerisierung

50 mg cyclo-(-D-Pro-Phe-Thr-Lys($CO(CH_2)_2COOH$)-Trp-Phe-) (55 µmol) werden in wenig DMF gelöst, mit 70 mg cyclo-(-D-Pro-Phe-Thr-Lys-Trp-Phe-) (86,8 µmol) und 21 mg DCC (100 µmol) versetzt. Man rührt 2 Tage bei Raumtemperatur. Die etwas eingeengte Reaktionsmischung wird an Sephadex LH20 / DMF chromatographiert.

Ausbeute: 45 mg = 48,3 % d.Th.
Smp.: 176 - 180 °C
DC: $R_{fA}$ 0,71 $R_{fB}$ 0,00 $R_{fC}$ 0,79

## Darstellung von dimeren Peptiden

### I CO-Brücke

0,1 mmol des Lysin-entschützten cyclischen Peptides werden in 1 ml Dimethylacetamid gelöst und auf -20 °C abgekühlt. Unter Rühren gibt man 12,2 mg (0,4 eq.) Bis(-4-nitrophenyl)carbonat hinzu, läßt die Reaktionsmischung auftauen und rührt noch 2 Tage bei RT. Anschließend wird eingeengt und das Peptidgemisch mit Wasser ausgefällt. Man filtriert ab, trocknet über Phosphorpentoxid und trennt monomere von dimeren Produkten durch Gelfiltration an Sephadex LH 20.
Ausbeute: um 50 %

### II Verbrückung durch Disäuren

0,04 mmol (1eq.) einer Disäure werden in 1 ml Dimethylacetamid gelöst. Man kühlt auf 0 °C ab und gibt unter Rühren 3 eq. EDCI (N-Ethyl-N'-(N,N-Dimethylaminopropyl)carbodiimid Hydrochlorid) (23 mg), 2,2 eq. Hydroxysuccinimid (10,1 mg) und 2,2 eq. N,N-Dimethylaminopyridin (10,7 mg) hinzu. Nach einer Stunde Rühren bei 0 °C bis Raumtemperatur werden 2,4 eq. des Peptides zugegeben, bevorzugt bei ca. -10 °C. Nach dem Auftauen des Kältebades rührt man noch zwei bis drei Tage bei Raumtemperatur. Aufarbeitung erfolgt entweder wie unter I beschrieben oder durch direkte Trennung des Reaktionsgemisches an Sephadex LH 20.
Ausbeute: je nach Disäure 40 - 80 % (bezogen auf Disäure)
Monomerfraktionen können recyclet werden.

Sulfateinführung in tyrosinhaltige Peptide ($-OSO_3Na$)

0,1 mmol des Lysin-geschützten Peptides werden bei Raumtemperatur in 1 ml abs. Pyridin gelöst. Man gibt 500 mg Pyridin-$SO_3$-Komplex hinzu und rührt 24 h. Zur Hydrolyse werden 20 ml dest. Wasser zugegeben; die Suspension trägt man auf eine Ionenaustauschersäule (mit 5 g (R)Lewatit CP 3050, Na-Form) auf und drückt mit Druckluft hindurch. Die erhaltene gelbliche Lösung wird ein zweites Mal mit dem gleichen Ionenaustauscher behandelt, diesmal ohne Druck. Danach destilliert man das Lösemittelgemisch bei Raumtemperatur vollständig ab. Aus dem verbleibenden Rückstand läßt sich das sulfatierte Peptid mit abs. Methanol extrahieren. Zur vollständigen Abtrennung anorganischer Salze ist jedoch ein zweiter Extraktionsschritt mit Filtration und Zentrifugation notwendig.
Rohausbeute: 92 %; Feinreinigung durch HPLC.

Phosphateinführung in tyrosinhaltige Peptide: ($-OPO_3Na_2$ od. $-OPO_3HNa$)

0,1 mmol Lysin-geschütztes Peptid werden in 2 ml abs. Pyridin gelöst. Bei -20 °C werden 46 mg (3 eq.) $POCl_3$ zugegeben. Man läßt auf 0 °C erwärmen und rührt noch 3 Stunden bei dieser Temperatur, zuletzt 10 min bei Raumtemperatur; gesamte Reaktionszeit: 4,5 h. Hydrolyse und Aufarbeitung wie oben beschrieben. Die Extraktion muß jedoch mit abs. Ethanol durchgeführt werden.
Rohausbeute: 75 %; Feinreinigung durch HPLC.

0248209
HOE 86/F 096 K

PATENTANSPRÜCHE

1. Verbindung der Formel I oder II

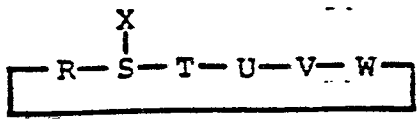

(I)

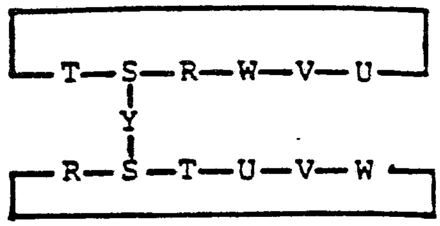

(II)

in der

R Thr, Val, Ala, Phe oder Thr, das gegebenenfalls verestert sein kann; T Trp;

S Lys, Orn, $C{\equiv}C-CH_2-NH_2$, $CH_2-C{\equiv}C-CH_2-NH_2$, $CO-(CH_2)_2-CO-O-(CH_2-CH_2-O)_m-CH_3$ oder $CO-(CH_2)_2-CO-O-(CH_2-CH_2-O)_m-H$;

U Phe, Phe(p-NHZ) oder Tyr, das ggf. verestert sein kann;

V D-Pro oder D-Ala;

W Phe oder Phe(p-NHZ);

X H, Z, $CO-C_6H_5$, $CO-C_6H_4(p-N_3)$ oder $CO-(CH_2)_n-C_6H_4(p-OSO_3Na)$;

Y $CO-(CH_2)_n-CO$, wobei max. 3 $CH_2$-Gruppen durch O ersetzt sein können, CO, $CO-C_6H_4(p-CO)$, $CO-CH_2-O-(CH_2-CH_2-O-)_m-CH_2-CO$ oder $CO-C{\equiv}C-CO$, $CO-(CH{=}CH)_n-CO$;

Z Benzyloxycarbonyl;

m eine ganze Zahl von 1 - 15 und

n eine ganze Zahl von 1 - 16

bedeuten,

sowie deren physiologisch verträglichen Salze.

2. Verbindung der Formel I oder II gemäß Anspruch 1, dadurch gekennzeichnet, daß mindestens einer der Substituenten bzw. Indices die folgende Bedeutung hat:

R Val oder Thr $(OSO_3Na)$,

U Tyr$(OSO_3Na)$,

Y $CO-(CH_2)_n-CO$, $CO-CH_2-O-(CH_2-CH_2-O)_m-CH_2-CO$ oder $CO-C{\equiv}C-CO$,

m eine ganze Zahl von 10-12 und

n 4.

3. Verfahren zur Herstellung einer Verbindung der Formel I und II gemäß Anspruch 1, dadurch gekennzeichnet, daß man ein lineares Hexapeptid einer der allgemeinen Formeln II a - II f

| | |
|---|---|
| $H - R - S(R^1) - T - U - V - W - OH$ | (II a) |
| $H - S(R^1) - T - U - V - W - R - OH$ | (II b) |
| $H - T - U - V - W - R - S(R^1) - OH$ | (II c) |
| $H - U - V - W - R - S(R^1) - T - OH$ | (II d) |
| $H - V - W - R - S(R^1) - T - U - OH$ | (II e) |
| $H - W - R - S(R^1) - T - U - V - OH$ | (II f) |

in denen R, S, T, U, V und W die in Anspruch 1 genannten Bedeutungen haben und $R^1$ für eine Schutzgruppe der δ- oder ε-Aminofunktion steht, nach bekannten Verfahren der Peptidsynthese cyclisiert, temporär eingeführte Schutzgruppen in geeigneter Weise abspaltet und anschließend zur Herstellung einer Verbindung der Formel II zwei solcherart erhaltene ungeschützte cyclische Hexapeptide der Formel I über eine Gruppe Y dimerisiert und das so erhaltene Peptid der Formel I oder II gegebenenfalls in sein physiologisch verträgliches Salz überführt.

4. Verfahren gemäß Anspruch 3, dadurch gekennzeichnet, daß man ein lineares Peptidderivat einer der Formel III a - III f

| | |
|---|---|
| $Boc - R - S(R^1) - T - U - V - W - NH-NH_2$ | (III a) |
| $Boc - S(R^1) - T - U - V - W - R - NH-NH_2$ | (III b) |
| $Boc - T - U - V - W - R - S(R^1) - NH-NH_2$ | (III c) |
| $Boc - U - V - W - R - S(R^1) - T - NH-NH_2$ | (III d) |
| $Boc - V - W - R - S(R^1) - T - U - NH-NH_2$ | (III e) |
| $Boc - W - R - S(R^1) - T - U - V - NH-NH_2$ | (III f) |

worin R, S, T, U, V, W und $R^1$ wie im Anspruch 3 definiert sind, nach Abspaltung der Boc-Gruppe über in situ hergestelltes Azid cyclisiert und anschließend die restlichen temporär eingeführten Schutzgruppen entfernt.

5. Verbindung einer der Formeln IVa - IVf

$R^2 - R - S(R^4) - T - U - V - W - R^3$ (IV a)

$R^2 - S(R^4) - T - U - V - W - R - R^3$ (IV b)

$R^2 - T - U - V - W - R - S(R^4) - R^3$ (IV c)

$R^2 - U - V - W - R - S(R^4) - T - R^3$ (IV d)

$R^2 - V - W - R - S(R^4) - T - U - R^3$ (IV e)

$R^2 - W - R - S(R^4) - T - U - V - R^3$ (IV f)

in denen R, S, T, U, V und W die in Anspruch 1 genannten Bedeutungen haben,

$R^2$ H, Z, Fmoc oder Boc,
$R^3$ OH oder $NH-NH_2$ bedeuten und
$R^4$ im Falle $R^2$ = H für Z oder Boc,
im Falle $R^2$ = Z oder Fmoc für Boc oder
im Falle $R^2$ = Boc für Z steht.

6. Verbindung der Formel I oder Formel II gemäß einem oder mehreren der Ansprüche 1 und 2 zur Verwendung als Heilmittel.

7. Heilmittel enthaltend eine Verbindung der Formel I oder Formel II gemäß einem oder mehreren der Ansprüche 1 und 2.

8. Verfahren zur Behandlung von akuten Läsionen der Leber, dadurch gekennzeichnet, daß man eine wirksame Menge einer Verbindung der Formel I oder II gemäß Anspruch 1 oder dessen physiologisch verträglichem Salz zusammen mit pharmazeutisch geeigneten Trägerstoffen appliziert.

9. Verfahren zur Behandlung von akuten Läsionen des exokrinen Pankreas, dadurch gekennzeichnet, daß man eine wirksame Menge einer Verbindung der Formel I oder II gemäß Anspruch 1 oder dessen physiologisch verträglichem Salz zusammen mit pharmazeutisch geeigneten Trägerstoffen appliziert.

10. Verfahren zur Behandlung von akuten Läsionen des Verdauungstraktes, dadurch gekennzeichnet, daß man eine wirksame Menge einer Verbindung der Formel I oder II gemäß Anspruch 1 oder dessen physiologisch verträglichem Salz zusammen mit pharmazeutisch geeigneten Trägerstoffen appliziert.

Patentansprüche Griechenland:

1. Verfahren zur Herstellung einer Verbindung der Formel I oder II

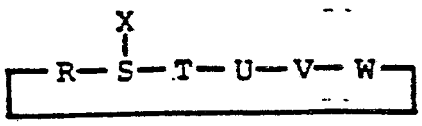

(I)

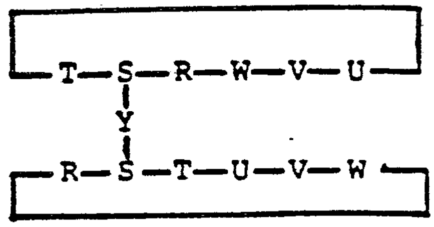

(II)

in der

R Thr, Val, Ala, Phe oder Thr, das gegebenenfalls verestert sein kann; T Trp;

S Lys, Orn, $C{\equiv}C-CH_2-NH_2$, $CH_2-C{\equiv}C-CH_2-NH_2$, $CO-(CH_2)_2-CO-O-(CH_2-CH_2-O)_m-CH_3$ oder $CO-(CH_2)_2-CO-O-(CH_2-CH_2-O)_m-H$;

U Phe, Phe(p-NHZ) oder Tyr, das ggf. verestert sein kann;

V D-Pro oder D-Ala;

W Phe oder Phe(p-NHZ);

X H, Z, $CO-C_6H_5$, $CO-C_6H_4(p-N_3)$ oder $CO-(CH_2)_n-C_6H_4(p-OSO_3Na)$;

Y $CO-(CH_2)_n-CO$, wobei max. 3 $CH_2$-Gruppen durch O ersetzt sein können, CO, $CO-C_6H_4(p-CO)$, $CO-CH_2-O-(CH_2-CH_2-O-)_m$ $CH_2-CO$ oder $CO-C{\equiv}C-CO$, $CO-(CH{=}CH)_n-CO$;

Z Benzyloxycarbonyl;

m eine ganze Zahl von 1 - 15 und

n eine ganze Zahl von 1 - 16

bedeuten,

sowie deren physiologisch verträglichen Salze, dadurch gekennzeichnet, daß man ein lineares Hexapeptid einer der allgemeinen Formeln IIa - IIf

| | |
|---|---|
| $H - R - S(R^1) - T - U - V - W - OH$ | (II a) |
| $H - S(R^1) - T - U - V - W - R - OH$ | (II b) |
| $H - T - U - V - W - R - S(R^1) - OH$ | (II c) |
| $H - U - V - W - R - S(R^1) - T - OH$ | (II d) |
| $H - V - W - R - S(R^1) - T - U - OH$ | (II e) |
| $H - W - R - S(R^1) - T - U - V - OH$ | (II f) |

in denen R, S, T, U, V und W die in Anspruch 1 genannten Bedeutungen haben und $R^1$ für eine Schutzgruppe der $\delta$- oder $\varepsilon$-Aminofunktion steht, nach bekannten Verfahren der Peptidsynthese cyclisiert, temporär eingeführte Schutzgruppen in geeigneter Weise abspaltet und anschließend zur Herstellung einer Verbindung der Formel II zwei solcherart erhaltene ungeschützte cyclische Hexapeptide der Formel I über eine Gruppe Y dimerisiert und das so erhaltene Peptid der Formel I oder II gegebenenfalls in sein physiologisch verträgliches Salz überführt.

2. Verfahren zur Herstellung einer Verbindung der Formel I oder II gemäß Anspruch 1, dadurch gekennzeichnet, daß mindestens einer der Substituenten die folgende Bedeutung hat:

R Val oder Thr($OSO_3Na$),
U Tyr($OSO_3Na$),
Y $CO-(CH_2)_n-CO$, $CO-CH_2-O-(CH_2-CH_2-O)_mCH_2-CO$ oder $CO-C{\equiv}C-CO$,
m eine ganze Zahl von 10-12 und
n 4.

3. Verfahren zur Herstellung einer Verbindung der Formel I oder II gemäß einem oder mehreren der Ansprüche 1 und 2, dadurch gekennzeichnet, daß man ein lineares Peptidderivat einer der Formel IIIa - IIIf

Boc - R - S($R^1$) - T - U - V - W - NH-$NH_2$ (III a)
Boc - S($R^1$) - T - U - V - W - R - NH-$NH_2$ (III b)
Boc - T - U - V - W - R - S($R^1$) - NH-$NH_2$ (III c)
Boc - U - V - W - R - S($R^1$) - T - NH-$NH_2$ (III d)
Boc - V - W - R - S($R^1$) - T - U - NH-$NH_2$ (III e)
Boc - W - R - S($R^1$) - T - U - V - NH-$NH_2$ (III f)

worin R, S, T, U, V, W und $R^1$ wie im Anspruch 1 definiert sind, nach Abspaltung der Boc-Gruppe über in situ hergestelltes Azid cyclisiert und anschließend die restlichen temporär eingeführten Schutzgruppen entfernt.

4. Verfahren zur Herstellung einer Verbindung einer der Formeln IVa - IVf

$R^2 - R - S(R^4) - T - U - V - W - R^3$ (IV a)
$R^2 - S(R^4) - T - U - V - W - R - R^3$ (IV b)
$R^2 - T - U - V - W - R - S(R^4) - R^3$ (IV c)
$R^2 - U - V - W - R - S(R^4) - T - R^3$ (IV d)
$R^2 - V - W - R - S(R^4) - T - U - R^3$ (IV e)
$R^2 - W - R - S(R^4) - T - U - V - R^3$ (IV f)

in denen R, S, T, U, V und W die in Anspruch 1 genannten Bedeutungen haben,

$R^2$ H, Z, Fmoc oder Boc,
$R^3$ OH oder $NH-NH_2$ bedeuten und
$R^4$ im Falle $R^2$ = H für Z oder Boc,
im Falle $R^2$ = Z oder Fmoc für Boc oder
im Falle $R^2$ = Boc für Z steht,

nach den in der Peptidsynthese bekannten Methoden.

5. Verbindung einer der Formeln IVa - IVf

$R^2 - R - S(R^4) - T - U - V - W - R^3$ (IV a)
$R^2 - S(R^4) - T - U - V - W - R - R^3$ (IV b)
$R^2 - T - U - V - W - R - S(R^4) - R^3$ (IV c)
$R^2 - U - V - W - R - S(R^4) - T - R^3$ (IV d)
$R^2 - V - W - R - S(R^4) - T - U - R^3$ (IV e)
$R^2 - W - R - S(R^4) - T - U - V - R^3$ (IV f)

in denen R, S, T, U, V und W die in Anspruch 1 genannten Bedeutungen haben,

$R^2$ H, Z, Fmoc oder Boc,
$R^3$ OH oder $NH-NH_2$ bedeuten und
$R^4$ im Falle $R^2$ = H für Z oder Boc,
im Falle $R^2$ = Z oder Fmoc für Boc oder
im Falle $R^2$ = Boc für Z steht.

Patentansprüche Österreich und Spanien:

1. Verfahren zur Herstellung einer Verbindung der Formel I oder II

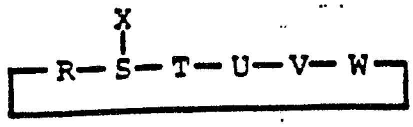

(I)

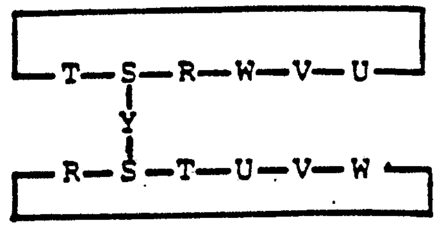

(II)

in der

R Thr, Val, Ala, Phe oder Thr, das gegebenenfalls verestert sein kann; T Trp;

S Lys, Orn, $C{\equiv}C{-}CH_2{-}NH_2$, $CH_2{-}C{\equiv}C{-}CH_2{-}NH_2$, $CO{-}(CH_2)_2{-}CO{-}O{-}(CH_2{-}CH_2{-}O)_m{-}CH_3$ oder $CO{-}(CH_2)_2{-}CO{-}O{-}(CH_2{-}CH_2{-}O)_m{-}H$;

U Phe, Phe(p-NHZ) oder Tyr, das ggf. verestert sein kann;

V D-Pro oder D-Ala;

W Phe oder Phe(p-NHZ);

X H, Z, $CO{-}C_6H_5$, $CO{-}C_6H_4(p{-}N_3)$ oder $CO{-}(CH_2)_n{-}C_6H_4(p{-}OSO_3Na)$;

Y $CO{-}(CH_2)_n{-}CO$, wobei max. 3 $CH_2$-Gruppen durch O ersetzt sein können, CO, $CO{-}C_6H_4(p{-}CO)$, $CO{-}CH_2{-}O{-}(CH_2{-}CH_2{-}O{-})_m{-}CH_2{-}CO$ oder $CO{-}C{\equiv}C{-}CO$, $CO{-}(CH{=}CH)_n{-}CO$;

Z Benzyloxycarbonyl;

m eine ganze Zahl von 1 - 15 und

n eine ganze Zahl von 1 - 16

bedeuten,

sowie deren physiologisch verträglichen Salze, dadurch gekennzeichnet, daß man ein lineares Hexapeptid einer der allgemeinen Formeln IIa - IIf

$H - R - S(R^1) - T - U - V - W - OH$ (II a)

$H - S(R^1) - T - U - V - W - R - OH$ (II b)

$H - T - U - V - W - R - S(R^1) - OH$ (II c)

$H - U - V - W - R - S(R^1) - T - OH$ (II d)

$H - V - W - R - S(R^1) - T - U - OH$ (II e)

$H - W - R - S(R^1) - T - U - V - OH$ (II f)

in denen R, S, T, U, V und W die in Anspruch 1 genannten Bedeutungen haben und $R^1$ für eine Schutzgruppe der $\delta$-oder $\varepsilon$-Aminofunktion steht, nach bekannten Verfahren der Peptidsynthese cyclisiert, temporär eingeführte Schutzgruppen in geeigneter Weise abspaltet und anschließend zur Herstellung einer Verbindung der Formel II zwei solcherart erhaltene ungeschützte cyclische Hexapeptide der Formel I über eine Gruppe Y dimerisiert und das so erhaltene Peptid der Formel I oder II gegebenenfalls in sein physiologisch verträgliches Salz überführt.

2. Verfahren zur Herstellung einer Verbindung der Formel I oder II gemäß Anspruch 1, dadurch gekennzeichnet, daß mindestens einer der Substituenten die folgende Bedeutung hat:

R Val oder Thr($OSO_3Na$),
U Tyr($OSO_3Na$),
Y $CO-(CH_2)_n-CO$, $CO-CH_2-O-(CH_2-CH_2-O)_mCH_2-CO$ oder $CO-C\equiv C-CO$,
m eine ganze Zahl von 10-12 und
n 4.

3. Verfahren zur Herstellung einer Verbindung der Formel I oder II gemäß einem oder mehreren der Ansprüche 1 und 2, dadurch gekennzeichnet, daß man ein lineares Peptidderivat einer der Formel IIIa - IIIf

Boc - R - S($R^1$) - T - U - V - W - $NH-NH_2$ (III a)
Boc - S($R^1$) - T - U - V - W - R - $NH-NH_2$ (III b)
Boc - T - U - V - W - R - S($R^1$) - $NH-NH_2$ (III c)
Boc - U - V - W - R - S($R^1$) - T - $NH-NH_2$ (III d)
Boc - V - W - R - S($R^1$) - T - U - $NH-NH_2$ (III e)
Boc - W - R - S($R^1$) - T - U - V - $NH-NH_2$ (III f)

worin R, S, T, U, V, W und $R^1$ wie im Anspruch 1 definiert sind, nach Abspaltung der Boc-Gruppe über in situ hergestelltes Azid cyclisiert und anschließend die restlichen temporär eingeführten Schutzgruppen entfernt.

4. Verfahren zur Herstellung einer Verbindung einer der Formeln IVa - IVf

$$R^2 - R - S(R^4) - T - U - V - W - R^3 \qquad \text{(IV a)}$$
$$R^2 - S(R^4) - T - U - V - W - R - R^3 \qquad \text{(IV b)}$$
$$R^2 - T - U - V - W - R - S(R^4) - R^3 \qquad \text{(IV c)}$$
$$R^2 - U - V - W - R - S(R^4) - T - R^3 \qquad \text{(IV d)}$$
$$R^2 - V - W - R - S(R^4) - T - U - R^3 \qquad \text{(IV e)}$$
$$R^2 - W - R - S(R^4) - T - U - V - R^3 \qquad \text{(IV f)}$$

in denen R, S, T, U, V und W die in Anspruch 1 genannten Bedeutungen haben,

$R^2$ H, Z, Fmoc oder Boc,
$R^3$ OH oder $NH-NH_2$ bedeuten und
$R^4$ im Falle $R^2$ = H für Z oder Boc,
im Falle $R^2$ = Z oder Fmoc für Boc oder
im Falle $R^2$ = Boc für Z steht,

nach den in der Peptidsynthese bekannten Methoden.